# EUROPEAN PATENT APPLICATION

(11) **EP 4 560 518 A1**
(43) Date of publication of application: **28.05.2025**
(21) Application number: 23929856.5
(22) Date of filing: 26.09.2023
(51) Int. Cl.: G06F 30/20, G16C 60/00, G06Q 10/04, G06F 119/10

(54) **PART RADIATION EMISSION ELECTROMAGNETIC FIELD RISK PREDICTION METHOD AND APPARATUS, DEVICE, AND MEDIUM**

(30) Priority: 29.03.2023 CN 202310320233
(71) Applicant: Deepal Automobile Nanjing Research Institute Co., Ltd., Nanjing, Jiangsu 211215 (CN)
(72) Inventor: TAN, Ruoxi, Chongqing 401135 (CN); YE, Shangbin, Chongqing 401135 (CN); DENG, Qingpeng, Chongqing 401135 (CN); HU, Jun, Chongqing 401135 (CN); ZHONG, Haibing, Chongqing 401135 (CN)
(74) Representative: 2K Patentanwälte Blasberg Kewitz & Reichel
(86) International application number: PCT/CN2023/121341
(87) International publication number: WO 2024/198282

(57) **Abstract**

The present invention provides a part radiation emission electromagnetic field risk prediction method and apparatus, a device, and a medium. The method comprises: acquiring part simulation data, wherein the part simulation data comprises physical device data and working parameter data; establishing a first simulation model on the basis of the part simulation data, and inputting actually measured common-mode current data into the first simulation model to carry out spatial domain solution so as to obtain a spatial domain electromagnetic field distribution and a port parameter; linking the spatial domain electromagnetic field distribution and the port parameter to a preset second simulation model for circuit simulation, and using actually measured noise data and the port parameter as input of the second simulation model to obtain a predicted field intensity value, so as to carry out risk prediction on a part radiation emission electromagnetic field. The method can improve the operation efficiency and the modeling precision, and realize the part radiation emission electromagnetic field risk prediction.

## Description

### TECHNICAL FIELD

The present invention relates to the field of electromagnetic radiation technologies, and in particular, to a part radiation emission electromagnetic field risk prediction method and apparatus, device, and medium.

### BACKGROUND

With a continuous rise in national resident income and upgrading of a consumption structure, families buy vehicles for convenient traveling and self-driving tours, driving the automotive industry to develop. There is a mandatory requirement on radiation emission performance of vehicles. With rapid development of the automotive industry, a failure of vehicle radiation emission to meet the requirement has become an obstacle to marketing and selling vehicles or devices of many enterprises. There are many components in a vehicle. The intensity of component radiation emission directly affects radiation emission of the vehicle.

In a conventional technology, in a component radiation emission evaluation method, usually, a test is completed in a test institution, and risk prediction and correction verification are performed by using simulation software. However, when the test is done in the test institution, there are problems such as a long test correction period, high costs, and tight test institution resources, and the test highly depends on an arrangement of a test institution consistency and testing personnel. Document CN105224741A discloses an electromagnetic radiation test planning method for a drive system of an electric vehicle. In this method, a simulation model needs to be calibrated based on test data, and then a quantity of test points is eliminated and reduced based on the simulation model, to obtain a test point position that reflects intensity of three-dimensional space radiation. Radiation emission is greatly affected by a test environment and a test rack layout. Therefore, calibration needs to be repeatedly performed for selection of test point positions at different test institutions, to ensure measurement precision. This increases time and costs of test calibration, reduces efficiency, and limits practicality of the method. Document CN112632823A discloses a simulation method and apparatus for predicting electromagnetic radiation. In this method, impact of a test antenna on a test result is considered, and a simulation application that performs simulation more matching an actual test condition is constructed. However, a frequency domain power source is used as an input of an excitation source into the model, and an actual frequency domain noise current or noise voltage has rich harmonic waves and a large data amount. If the excitation is directly imported into finite element simulation software, a calculation amount of the model is large, a device running memory requirement is very high, calculation time is long, and efficiency is low. Due to high calculation costs, it is very difficult to cover full-band interference source features. Consequently, precision and calculation efficiency of simulation prediction are severely limited. Therefore, if simulation calculation is performed by using simulation software used in the conventional technology, there are problems such as relatively great impact of the test environment and the test rack layout, long test point selection time, high test costs, a large calculation amount, long calculation time, and low efficiency.

### SUMMARY

In view of the foregoing disadvantages of the conventional technology, the present invention provides a component radiation emission electromagnetic field risk prediction method and apparatus, a device, and a medium, to resolve the foregoing technical problems of low efficiency and low accuracy of component radiation emission electromagnetic field risk prediction.

Other features and advantages of the present invention become clear in the following detailed descriptions, or may be partially obtained by practice of the present invention.

According to an aspect of an embodiment of the present invention, a component radiation emission electromagnetic field risk prediction method is provided, including: obtaining component simulation data, where the component simulation data includes physical device data and working parameter data; building a first simulation model based on the component simulation data, and inputting actually measured common mode current data into the first simulation model to perform spatial domain solution, to obtain a spatial domain electromagnetic field distribution and a port parameter; and linking the spatial domain electromagnetic field distribution and the port parameter to a preset second simulation model used to perform circuit simulation, and using measured noise data and the port parameter as inputs into the second simulation model, to obtain a predicted field strength value, so as to perform risk prediction on a component radiation emission electromagnetic field.

According to an aspect of an embodiment of the present invention, a component radiation emission electromagnetic field risk prediction apparatus is provided, including: an obtaining module, configured to obtain component simulation data, where the component simulation data includes physical device data and working parameter data; a first simulation module, configured to: build a first simulation model based on the component simulation data, and input actually measured common mode current data into the first simulation model to perform spatial domain solution, to obtain a spatial domain electromagnetic field distribution and a port parameter; and a second simulation module, configured to: link the spatial domain electromagnetic field distribution and the port parameter to a preset second simulation model used to perform circuit simulation, and use measured noise data and the port parameter as inputs into the second simulation model, to obtain a predicted field strength value, so as to perform risk prediction on a component radiation emission electromagnetic field.

In an embodiment of the present invention, based on the foregoing solution, the first simulation module includes: a first simulation physical module, configured to: set a material attribute of each constituent part of a component based on the physical device data, and build a first simulation physical model; and a first simulation port module, configured to: set a simulation port parameter based on the working parameter data, and build a first simulation port model; and build the first simulation model based on the first simulation physical model and the first simulation port model.

In an embodiment of the present invention, based on the foregoing solution, the first simulation port module is configured to: set a component high-voltage output port to a first simulation port, set a component low-voltage output port to a second simulation port, set a high-voltage LISN port to a third simulation port, set a low-voltage LISN port to a fourth simulation port, and set a fifth simulation port between an antenna and a copper ground plate, to obtain the first simulation port model.

In an embodiment of the present invention, based on the foregoing solution, the second simulation module is configured to: input the actually measured common mode current data into the first simulation model to perform spatial domain solution, to obtain the spatial domain electromagnetic field distribution and the port parameter; and based on the port parameter, input an actually measured high-voltage current noise source into a first circuit port, input an actually measured low-voltage current noise source into a second circuit port, input a high-voltage LISN into a third circuit port, input a low-voltage LISN into a fourth circuit port, and ground a fifth circuit port to output a predicted field strength value.

In an embodiment of the present invention, based on the foregoing solution, a common mode current on a high voltage direct current cable of the component and a common mode current on a low voltage harness are measured, to obtain the common mode current data; and the common mode current data is used as an initial excitation source of the first simulation model, and is input into the first simulation model.

In an embodiment of the present invention, based on the foregoing solution, the first simulation model includes a component three-dimensional equivalent model, a high voltage direct current cable equivalent model, a low voltage harness equivalent model, a high-voltage LISN equivalent model, a low-voltage LISN equivalent model, a test bench equivalent model, a ground plate equivalent model, and a receive antenna equivalent model.

In an embodiment of the present invention, based on the foregoing solution, before the measured noise data and the port parameter are used as inputs into the second simulation model, to obtain the predicted field strength value, the following operations are further included: setting a frequency domain calculation range, and comparing the frequency domain calculation range with an actually measured excitation source; resetting the frequency domain calculation range if the frequency domain calculation range is inconsistent with the actually measured excitation source, and determining that the frequency domain calculation range is set correctly if the frequency domain calculation range is consistent with the actually measured excitation source.

In an embodiment of the present invention, based on the foregoing solution, using measured noise data and the port parameter as inputs into the second simulation model is configured as: performing simplification of the measured noise data, to obtain simplified data; obtaining new measured noise data to perform the simplification if an interference feature of the simplified data is inconsistent with an interference feature of the measured noise data; and inputting the simplified data into the second simulation model if the interference feature of the simplified data is consistent with the interference feature of the measured noise data.

In an embodiment of the present invention, based on the foregoing solution, radiation emission data is obtained at a distance of a first preset value from a component test rack; the measured noise data and the port parameter are used as an excitation source of the second simulation model, to obtain the predicted field strength value; and comparison verification is performed on the radiation emission data and the predicted field strength value, to perform risk prediction on the component radiation emission electromagnetic field.

According to an aspect of an embodiment of the present invention, an electronic device is provided. The electronic device includes: one or more processors; and a storage apparatus, configured to store one or more programs. When the one or more programs are executed by the one or more processors, the electronic device is enabled to implement the component radiation emission electromagnetic field risk prediction method in any one of the foregoing embodiments.

The present invention further provides a computer-readable storage medium. The computer-readable storage medium stores a computer program, and when the computer program is executed by a processor of a computer, the computer is enabled to perform the component radiation emission electromagnetic field risk prediction method in any one of the foregoing embodiments.

Beneficial effects of the present invention are as follows: the present invention provides a component radiation emission electromagnetic field risk prediction method and apparatus, a device, and a medium. In the present invention, problems of a huge calculation amount and low calculation efficiency caused by directly importing a frequency domain interference source into finite element simulation software are avoided, and frequency domain calculation is performed in circuit simulation software without involving division into grids, thereby reducing a calculation amount, and improving calculation efficiency. In addition, precision of the simulation model built in this method is not affected by a change in a test environment. The model has universality and relatively good engineering practicality, and is flexible to operate. Therefore, test costs can be reduced, a correction period of a nonconforming product is shortened, and component electromagnetic compatibility evaluation efficiency is greatly improved.

It should be understood that the foregoing general descriptions and the following detailed descriptions are merely examples and explanations, and cannot limit the present invention.

### BRIEF DESCRIPTION OF DRAWINGS

The accompanying drawings herein are incorporated in this specification, become a part of this specification, show embodiments that are in accordance with this application, and are used with this specification to explain a principle of this application. Clearly, the accompanying drawings in the following descriptions are merely some embodiments of this application. A person of ordinary skill in the art may still derive other drawings from these accompanying drawings without creative efforts. In the accompanying drawings:
FIG. 1 is a schematic diagram of an example system architecture that can apply a technical solution of an embodiment of the present invention;
FIG. 2 is a schematic flowchart of a component radiation emission electromagnetic field risk prediction method according to an embodiment of the present invention;
FIG. 3 shows a component test layout according to an embodiment of the present invention;
FIG. 4 is a schematic flowchart of building a first simulation model according to an embodiment of the present invention;
FIG. 5 is a schematic diagram of a radiation emission simulation model according to an embodiment of the present invention;
FIG. 6 is a schematic diagram of a port equivalent circuit model according to an embodiment of the present invention;
FIG. 7 shows actually measured frequency domain common mode current data according to an embodiment of the present invention;
FIG. 8 is a diagram of a comparison between a predicted field strength value and actually measured radiation emission data according to an embodiment of the present invention;
FIG. 9 is a block diagram of a component radiation emission electromagnetic field risk prediction apparatus according to an embodiment of the present invention; and
FIG. 10 is a schematic structural diagram of a computer system suitable for implementing an electronic device according to an embodiment of the present invention.

1: Semi-anechoic chamber; 2: Metal ground plane; 3: Low dielectric constant support; 4: Low voltage harness; 5: High voltage direct current cable; 6: Component assembly; 7: Ground cable; 8: Mechanical connecting shaft; 9: Filtering mechanical bearing; 10: Dynamometer; 11: Simulator monitoring device; 12: Wall board connector; 13: High-voltage LISN; 14: Low-voltage LISN; 15: Load resistor 50Ω; 16: 12V battery; 17: High voltage power supply; 18: Coaxial feeder; 19: Receive antenna; 20: Antenna connection metal plate; 21: Ground connecting bar; 22: Low-voltage current probe; 23: High-voltage current probe; and 24: Receiver.

### DESCRIPTION OF EMBODIMENTS

The following describes implementations of the present invention with reference to accompanying drawings and preferred embodiments. A person skilled in the art can easily understand other advantages and effects of the present invention based on content disclosed in this specification. The present invention may be further implemented or applied by using other different specific implementations. Various details in this specification may also be modified or changed based on different viewpoints and applications without departing from the present invention. It should be understood that the preferred embodiments are merely intended to describe the present invention, and are not intended to limit the protection scope of the present invention.

It should be noted that the drawings provided in the following embodiments merely describe the basic concept of the present invention by using examples. Although the drawings show only components related to the present invention, and are not drawn based on a quantity of components, a shape of a component, and a size of a component during actual implementation, a shape, a quantity, and a scale of the components may be arbitrarily changed during actual implementation, and a component layout form may be more complex.

In the following descriptions, a large quantity of details are discussed to provide a more thorough explanation of the embodiments of the present invention. However, it is clear to a person skilled in the art that the embodiments of the present invention may be implemented without these specific details. In other embodiments, well-known structures and devices are shown in a block diagram rather than in a detail form, to avoid making the embodiments of the present invention difficult to understand.

First, it should be noted that an electric drive system is a drive system in which a motor serves as a power energy source. An electric drive system of a modern electric vehicle mainly includes four parts: a drive motor, a transmission, a power converter, and a controller. The drive motor is a core of the electric drive system, and performance and efficiency of the drive motor directly affect performance of the electric vehicle. A size and a weight of the drive motor and a size and a weight of the transmission also affect overall efficiency of the vehicle. The power converter and the controller are closely related to safe and reliable operation of the electric vehicle. The electric drive system is a crucial mechanism in the electric vehicle, and directly affects a vehicle lifetime and driver safety.

A simulation technology is a simulation model technology in which a behavior or a process of a system is reflected by applying simulation hardware and simulation software through a simulation experiment and some numerical calculation and problem solution. The simulation technology is combined with a network technology, and a combined simulation system of a real device and a computer simulation system forms a simulation environment, to restore and test a real situation, and discover and solve a problem in a timely manner.

A spatial domain is also referred to as image space, namely, space including image pixels. Directly processing a pixel value in the image space by using a length or a distance as an independent variable is referred to as spatial domain processing.

Radiation emission is a phenomenon in which electromagnetic radiation is generated after a substance absorbs energy, and is essentially a radiative transition. To be specific, after a particle of the substance absorbs energy to be excited to a high energy state, the particle returns to a ground state or a low energy state instantly, and excess energy is released in a form of electromagnetic radiation. There are two basic types of radiation emission: differential mode radiation and common mode radiation. Common mode radiation or monopole antenna radiation is caused by an unintentional voltage drop, and raises potentials of all ground connections in a circuit above a system ground potential. In terms of a magnitude of an electric field, common mode radiation is more serious than differential mode radiation.

Finite element analysis is to simulate a real physical system (geometry and load conditions) through mathematical approximation. Elements that are simple and interact with each other are used, to approximate a real system with an infinite quantity of unknown quantities by using a limited quantity of unknown quantities. Finite element analysis is to replace a complex problem with a simple problem and then find a solution. A solution domain is considered to include many small interconnected subdomains referred to as finite elements, a proper approximate solution is assumed for each unit, and then a total condition that needs to be met to solve the domain is derived, to obtain a solution to the problem. This solution is not an accurate solution, but an approximate solution, because the actual problem is replaced by the relatively simple problem. Because it is difficult to find an accurate solution to most actual problems, and a finite element not only has high calculation precision, but also can adapt to various complex shapes, the finite element becomes an effective engineering analysis means.

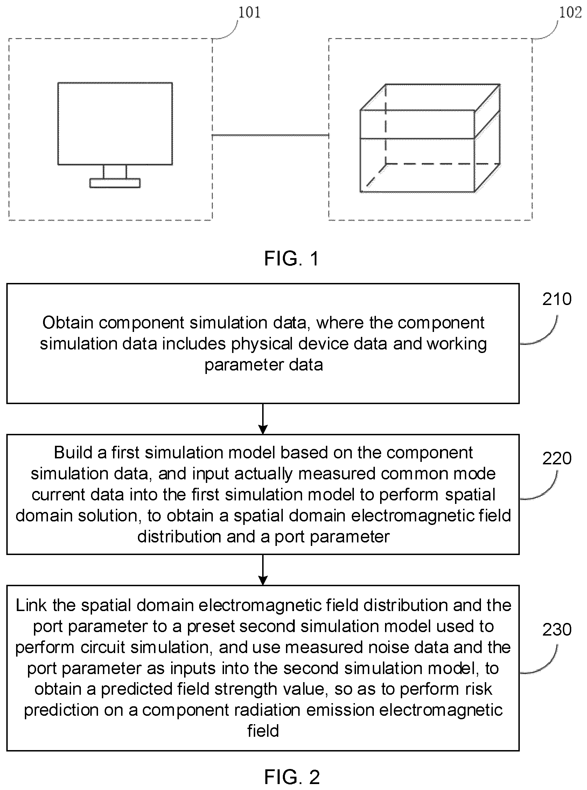

FIG. 1 is a schematic diagram of an example system architecture that can be applied to a technical solution of an embodiment of the present invention. As shown in FIG. 1, the system architecture may include a computer device 101 and a component radiation emission electromagnetic field risk prediction apparatus 102. The computer device may be at least one of a general-purpose computer, a neural network computer, or the like. A related technician may use the computer device 101 to perform simulation calculation on radiation emission data of a to-be-tested component, to obtain a component electromagnetic radiation emission risk prediction result. The component radiation emission electromagnetic field risk prediction apparatus 102 is configured to: collect the radiation emission data of the to-be-tested component, and provide the radiation emission data to the computer device 101 for processing, to implement component electromagnetic radiation emission risk prediction.

For example, after obtaining component simulation data, the computer device 101 builds a first simulation model based on the component simulation data, inputs actually measured common mode current data into the first simulation model to perform spatial domain solution, to obtain a spatial domain electromagnetic field distribution and a port parameter; and links the spatial domain electromagnetic field distribution and the port parameter to a preset second simulation model used to perform circuit simulation, and uses measured noise data and the port parameter as inputs into the second simulation model, to obtain a predicted field strength value, so as to perform risk prediction on a component radiation emission electromagnetic field. The component simulation data includes physical device data and working parameter data. In an embodiment of the present invention, a field strength value prediction result of the to-be-tested component may be obtained by using a radiation emission simulation model and a port equivalent circuit model, to improve accuracy of the prediction result and prediction efficiency.

To resolve the foregoing technical problems, the present invention provides a component radiation emission electromagnetic field risk prediction method and apparatus, a device, and a medium. The following describes in detail implementation details of the technical solutions in the embodiments of the present invention.

FIG. 2 is a schematic flowchart of a component radiation emission electromagnetic field risk prediction method according to an embodiment of the present invention. As shown in FIG. 2, in an example embodiment, the component radiation emission electromagnetic field risk prediction method includes at least step S210 to step S230. Detailed descriptions are as follows:
Step S210: Obtain component simulation data, where the component simulation data includes physical device data and working parameter data.
   In an embodiment of the present invention, the physical device data includes a material attribute parameter of each constituent part of a component and a size, a shape, and a distance of the constituent part; and the working parameter data includes a port parameter of the component. The component simulation data may be entered into a computer device 101 by a related technician.
Step S220: Build a first simulation model based on the component simulation data, and input actually measured common mode current data into the first simulation model to perform spatial domain solution, to obtain a spatial domain electromagnetic field distribution and a port parameter.

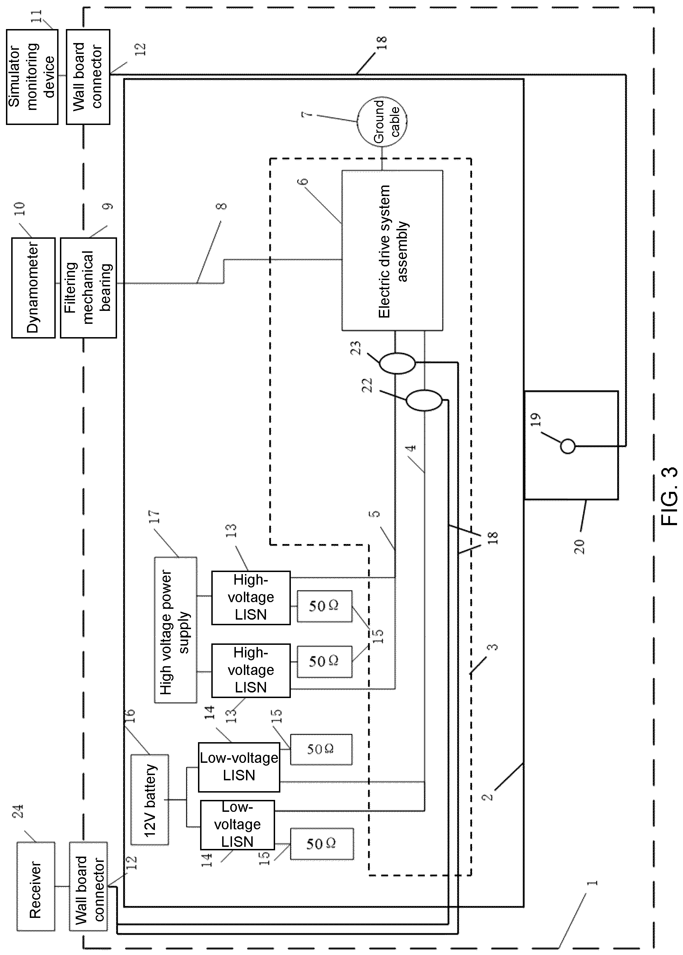

   FIG. 3 shows a component test layout according to an embodiment of the present invention. As shown in FIG. 3, in an example embodiment, when the component is an electric drive system, the component simulation data includes component test three-dimensional layout data. A component assembly 6 is an electric drive system assembly 6. In finite element simulation software, the material attribute is set for each constituent part of the component based on the physical device data in the component simulation data, a simulation port parameter is set for a component port based on the working parameter data in the component simulation data, and a radiation emission simulation model is built. A frequency domain range of simulation calculation is set, for example, is set to 150 kHz to 30 MHz, voltage excitation is set, a spatial domain is set, and a radiation boundary condition is set on a surface of the spatial domain. The spatial domain is set with reference to a requirement of a standard anechoic chamber of a test institution. The actually measured common mode current data is input into the first simulation model to perform spatial domain solution, to obtain the spatial domain electromagnetic field distribution and the port parameter.
Step S230: Link the spatial domain electromagnetic field distribution and the port parameter to a preset second simulation model used to perform circuit simulation, and use measured noise data and the port parameter as inputs into the second simulation model, to obtain a predicted field strength value, so as to perform risk prediction on a component radiation emission electromagnetic field.

In an embodiment of the present invention, in circuit simulation software, the spatial domain electromagnetic field distribution and the port parameter are linked to a preset port equivalent circuit model. The port equivalent circuit model is used to perform circuit simulation. A frequency domain calculation range is set. The frequency domain range needs to be consistent with an actually measured excitation source. The measured noise data and the port parameter are used as the excitation source, and are input into the port equivalent circuit model, to calculate a predicted field strength value received through an antenna port. This avoids problems of a huge calculation amount and low calculation efficiency caused by directly importing a frequency domain interference source into the finite element simulation software, and frequency domain calculation is performed in the circuit simulation software without involving division into grids, to improve calculation efficiency.

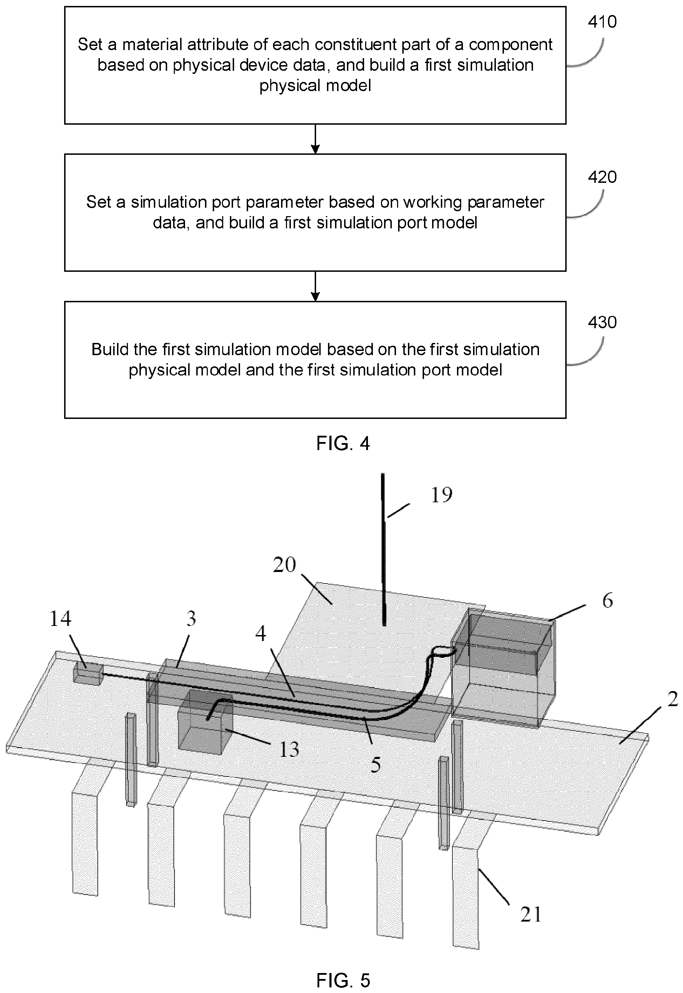

FIG. 4 is a schematic flowchart of building a first simulation model according to an embodiment of the present invention. As shown in FIG. 4, in an example embodiment, the building a first simulation model based on the component simulation data includes at least step S410 to step S430. Detailed descriptions are as follows:

Step S410: Set the material attribute of each constituent part of the component based on the physical device data, and build a first simulation physical model.

In an embodiment of the present invention, that the component is an electric drive system is used as an example. Material attributes of housings of an electric drive, a high-voltage LISN, and a low-voltage LISN are all set to aluminum; material attributes of a high voltage cable and a low voltage harness are set to copper; a material attribute of a 50 mm thick insulation pad on which the high voltage cable and low voltage harness are placed is set to foam; a material attribute of a test bench is set to wood; a material attribute of a ground plate on the table is set to copper; a material attribute of a grounding copper bar material connecting the ground plate and a spatial domain ground is set to a perfect electrical conductor; and a spatial domain represents an anechoic chamber in a test environment. Therefore, a spatial domain bottom is set to a ground surface, and a material attribute thereof is set to a perfect electrical conductor, to form good grounding with the ground plate on the test bench. A material attribute of a receive antenna is set to a perfect electrical conductor attribute, and a material attribute of a support plate connected to the antenna is set to an aluminum plate. The support plate is directly in contact with the ground, to form good grounding, which is highly consistent with the test environment.

Step S420: Set the simulation port parameter based on the working parameter data, and build a first simulation port model.

In an embodiment of the present invention, that the component is an electric drive system is used as an example. The simulation port parameter is set based on the working parameter data, and the first simulation port model is built. Specifically, an electrical drive high voltage output port is set as a first simulation port, an electrical drive low voltage output port is set as a second simulation port, a high-voltage LISN port is set as a third simulation port, a low-voltage LISN port is set as a fourth simulation port, and a fifth simulation port is set between the antenna and a copper ground plate, to obtain the first simulation port model.

In this embodiment, a lumped port first simulation port Lumport 1 is set at a port that is close to an electric drive and that is of a high voltage direct current cable that is of the electric drive and that is connected to a battery pack, a second simulation port Lumport 2 is set at the electric drive low voltage output port, a third simulation port Lumport 3 is added at a high-voltage LISN port that is connected between the battery pack and the high voltage direct current cable of the electric drive and that is close to the battery pack, a fourth simulation port Lumport 4 is added at a low-voltage LISN port that is connected between a 12V battery and a low voltage harness of the electric drive and that is close to the battery, and a lumped port fifth simulation port Lumport 5 is added between a pole antenna and the copper ground plate.

Step S430: Build the first simulation model based on the first simulation physical model and the first simulation port model. FIG. 5 shows a three-dimensional simulation graph corresponding to FIG. 3.

In an embodiment of the present invention, the radiation emission simulation model is built based on the first simulation physical model having the material attribute of each constituent part of the component and the first simulation port model having each simulation port parameter. Precision of the built radiation emission simulation model is not affected by a change in the test environment. The model has universality and relatively good engineering practicality. Therefore, test costs can be reduced, a correction period of a nonconforming product is shortened, and component electromagnetic compatibility evaluation efficiency is improved.

It should be noted that there is no strict sequence between step S410 and step S420 in FIG. 4. To be specific, step S410 may be performed before step S420, or step S420 may be performed before step S410, or step S410 and step S420 are performed simultaneously.

In an embodiment of the present invention, after the first simulation port model is obtained, the actually measured common mode current data is input into the first simulation model to perform spatial domain solution, to obtain the spatial domain electromagnetic field distribution and the port parameter; and based on the port parameter, an actually measured high-voltage current noise source is input into a first circuit port, an actually measured low-voltage current noise source is input into a second circuit port, a high-voltage LISN is input into a third circuit port, a low-voltage LISN is input into a fourth circuit port, and a fifth circuit port is grounded to output a predicted field strength value.

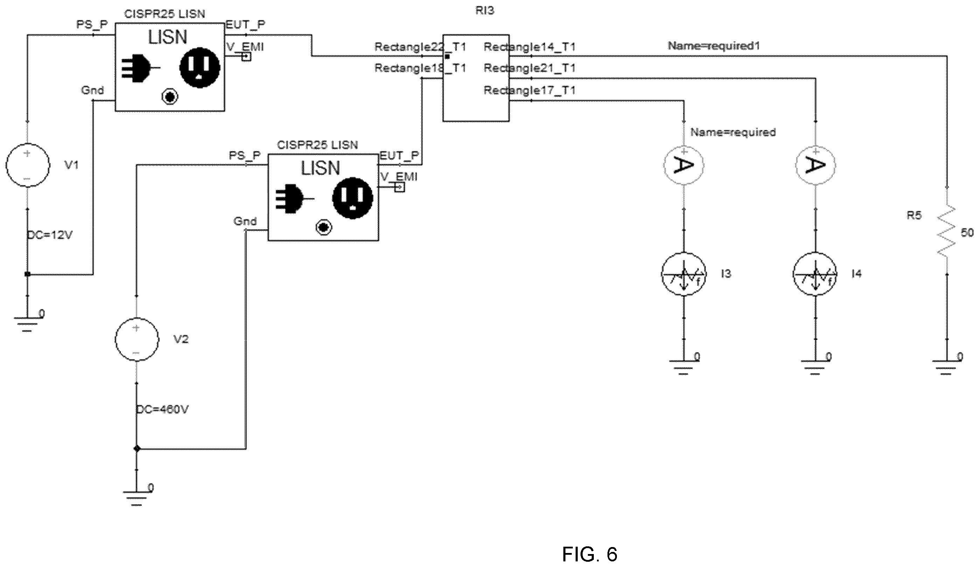

In an embodiment of the present invention, after the radiation emission simulation model is obtained, the calculated radiation emission simulation model is directly linked to the port equivalent circuit model. As shown in FIG. 6, the port equivalent circuit model includes five ports: a first circuit port Lumport 01, a second circuit port Lumport 02, a third circuit port Lumport 03, a fourth circuit port Lumport 04, and a fifth circuit port Lumport 05. The actually measured high-voltage current noise source is added to the first circuit port Lumport 01; the actually measured low-voltage current noise source is added to the second circuit port Lumport 02; the high-voltage LISN is selected in the circuit simulation software, and the third circuit port Lumport 03 is connected to an input end of the high-voltage LISN; similarly, the fourth circuit port Lumport 04 is connected to an input end of the low-voltage LISN; and the fifth circuit port Lumport 05 is connected to a 50 ohms resistor and is grounded.

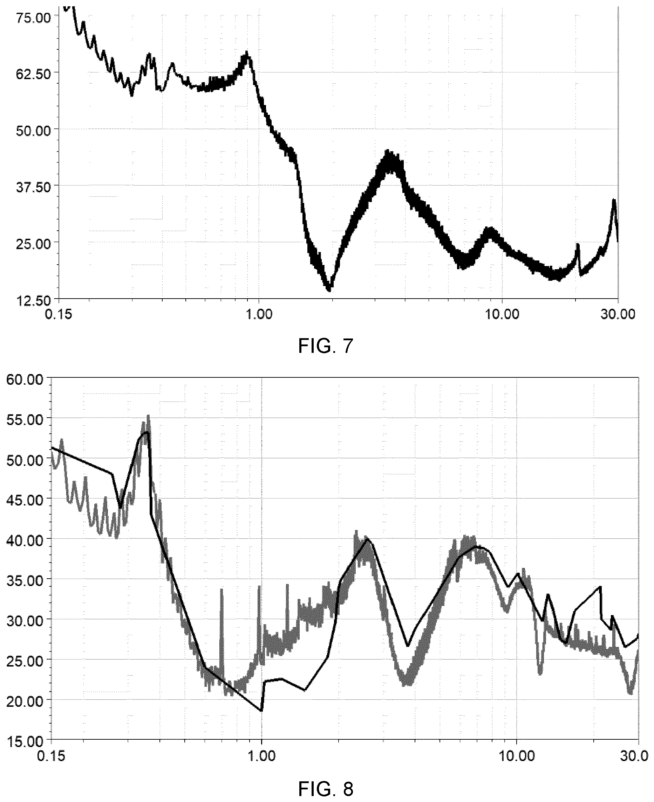

In an embodiment of the present invention, the component is controlled to normally work with load, a current clamp is used to separately measure a common mode current I_{CM} on the high voltage direct current cable and a common mode current I_{cm} on the low voltage harness, and a receiver is used to obtain high-voltage frequency domain common mode current data and low-voltage frequency domain common mode current data. As shown in FIG. 7, an excitation source serving as the radiation emission simulation model is input to the radiation emission simulation model. In FIG. 7, a horizontal coordinate represents a frequency, and a vertical coordinate represents a high-voltage common mode current I_{CM}.

In an embodiment of the present invention, the first simulation model, namely, the radiation emission simulation model includes a component three-dimensional equivalent model, a high voltage direct current cable equivalent model, a low voltage harness equivalent model, a high-voltage LISN equivalent model, a low-voltage LISN equivalent model, a test bench equivalent model, a ground plate equivalent model, and a receive antenna equivalent model.

In an embodiment of the present invention, before the measured noise data and the port parameter are used as inputs into the second simulation model, to obtain the predicted field strength value, the frequency domain calculation range needs to be set, and the frequency domain range needs to be consistent with the actually measured excitation source. The frequency domain calculation range is reset if the frequency domain range is inconsistent with the actually measured excitation source.

In an embodiment of the present invention, the measured noise data and the port parameter are used as inputs into the second simulation model; the measured noise data is simplified based on an interference feature of the noise data; if an interference feature of the simplified data obtained through simplification is inconsistent with the interference feature of the measured noise data, new measured noise data is obtained to perform simplification; and the simplified data is input into a data input port equivalent circuit model if the interference feature of the simplified data is consistent with the interference feature of the measured noise data. Simplification may be performed by using MATLAB (Matrix Laboratory), or may be performed in an envelope extraction method of Excel (spreadsheet). A simplification method is not limited herein. Simplification reduces a calculation amount and improves calculation efficiency.

In an embodiment of the present invention, radiation emission data at a distance of a first preset value from a component test rack is obtained through the receive antenna. For example, radiation emission data at a distance of one meter from the component test rack is obtained. A comparison between the radiation emission data and the predicted field strength value is performed, and is used as a reference basis for verifying consistency between simulation and actual measurement.

In an embodiment of the present invention, a field strength value extracted through simulation is compared with an actually measured data value, to obtain a diagram of a comparison between the predicted field strength value and the actually measured radiation emission data, as shown in FIG. 8. It can be learned from a simulation result that the predicted field strength value obtained by the model is highly consistent with the actually measured radiation emission data, and therefore, a component radiation emission risk can be predicted efficiently and accurately. In FIG. 8, a horizontal coordinate represents a frequency, a vertical coordinate represents electromagnetic field strength, a gray curve represents the actually measured radiation emission data, and a black curve represents the predicted field strength value.

In an embodiment of the present invention, the component may alternatively be a compressor and a generator. Component simulation data of the compressor or the generator is obtained. The component simulation data includes physical device data and working parameter data. A first simulation model is built based on the component simulation data, and actually measured common mode current data is input into the first simulation model to perform spatial domain solution, to obtain a spatial domain electromagnetic field distribution and a port parameter. The spatial domain electromagnetic field distribution and the port parameter are linked to a preset second simulation model used to perform circuit simulation, and measured noise data and the port parameter are used as inputs into the second simulation model, to obtain a predicted field strength value, so as to perform risk prediction on a component radiation emission electromagnetic field. It should be noted that this embodiment and the component radiation emission electromagnetic field risk prediction method provided in the foregoing embodiment belong to a same concept. Some specific manners in which an operation is performed are described in detail in the foregoing embodiment. Details are not described herein again.

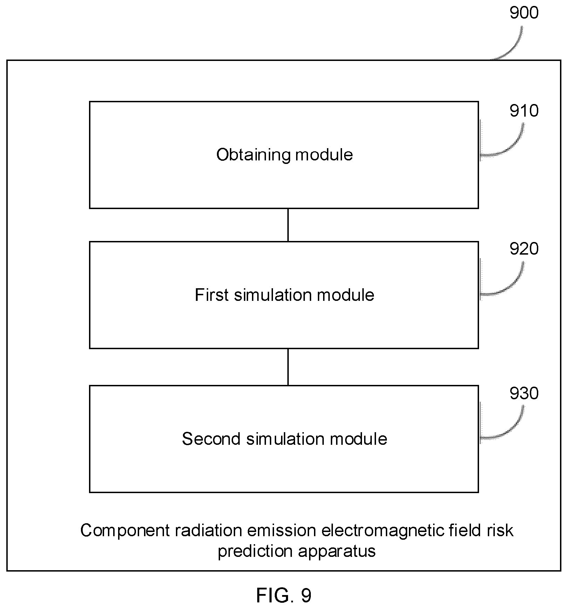

FIG. 9 is a block diagram of a component radiation emission electromagnetic field risk prediction apparatus according to an embodiment of the present invention. The apparatus may be applied to an implementation environment shown in FIG. 1, and is specifically configured in a computer device 101. The apparatus may also be applied to another example implementation environment, and is specifically configured in another device. This embodiment sets no limitation on an implementation environment to which the apparatus is applicable.

As shown in FIG. 9, a component radiation emission electromagnetic field risk prediction apparatus 900 according to an embodiment of the present invention includes an obtaining module 910, a first simulation module 920, and a second simulation module 930.

The obtaining module 910 is configured to obtain component simulation data. The component simulation data includes physical device data and working parameter data. The first simulation module 920 is configured to: build a first simulation model based on the component simulation data, and input actually measured common mode current data into the first simulation model to perform spatial domain solution, to obtain a spatial domain electromagnetic field distribution and a port parameter. The second simulation module 930 is configured to: link the spatial domain electromagnetic field distribution and the port parameter to a preset second simulation model used to perform circuit simulation, and use measured noise data and the port parameter as inputs into the second simulation model, to obtain a predicted field strength value, so as to perform risk prediction on a component radiation emission electromagnetic field.

In an embodiment of the present invention, based on the foregoing solution, the first simulation module 920 includes: a first simulation physical module, configured to set a material attribute of each constituent part of a component based on the physical device data; and a first simulation port module, configured to set a simulation port parameter based on the working parameter data.

In an embodiment of the present invention, based on the foregoing solution, the first simulation port module is configured to: set a component high-voltage output port to a first simulation port, set a component low-voltage output port to a second simulation port, set a high-voltage LISN port to a third simulation port, set a low-voltage LISN port to a fourth simulation port, and set a fifth simulation port between an antenna and a copper ground plate, to obtain a first simulation port model.

In an embodiment of the present invention, based on the foregoing solution, the second simulation module 930 is configured to: input the actually measured common mode current data into the first simulation model to perform spatial domain solution, to obtain the spatial domain electromagnetic field distribution and the port parameter; and based on the port parameter, input an actually measured high-voltage current noise source into a first circuit port, input an actually measured low-voltage current noise source into a second circuit port, input a high-voltage LISN into a third circuit port, input a low-voltage LISN into a fourth circuit port, and ground a fifth circuit port to output a predicted field strength value.

In an embodiment of the present invention, based on the foregoing solution, a common mode current on a high voltage direct current cable of the component and a common mode current on a low voltage harness are measured; and the measured common mode current data is used as an initial excitation source of the first simulation model, and is input into the first simulation model.

In an embodiment of the present invention, based on the foregoing solution, the first simulation model 920 includes a component three-dimensional equivalent model, a high voltage direct current cable equivalent model, a low voltage harness equivalent model, a high-voltage LISN equivalent model, a low-voltage LISN equivalent model, a test bench equivalent model, a ground plate equivalent model, and a receive antenna equivalent model.

In an embodiment of the present invention, based on the foregoing solution, before the measured noise data and the port parameter are used as inputs into the second simulation model, to obtain the predicted field strength value, the following operations are further included: setting a frequency domain calculation range, and comparing the frequency domain calculation range with an actually measured excitation source; resetting the frequency domain calculation range if the frequency domain calculation range is inconsistent with the actually measured excitation source, and determining that the frequency domain calculation range is set correctly if the frequency domain calculation range is consistent with the actually measured excitation source.

In an embodiment of the present invention, based on the foregoing solution, the using measured noise data and the port parameter as inputs into the second simulation model is configured as: simplifying the measured noise data, to obtain simplified data; if an interference feature of the simplified data is inconsistent with an interference feature of the measured noise data, obtaining new measured noise data to perform simplification; and inputting the simplified data into the second simulation model if an interference feature of the simplified data is consistent with an interference feature of the measured noise data.

In an embodiment of the present invention, based on the foregoing solution, radiation emission data is obtained at a distance of a first preset value from a component test rack; the measured noise data and the port parameter are used as an excitation source of the second simulation model, to obtain the predicted field strength value; and comparison verification is performed on the radiation emission data and the predicted field strength value, to perform risk prediction on the component radiation emission electromagnetic field.

It should be noted that the component radiation emission electromagnetic field risk prediction apparatus provided in the foregoing embodiment and the component radiation emission electromagnetic field risk prediction method provided in the foregoing embodiment belong to a same concept. A specific manner in which each module and unit perform an operation is described in detail in the method embodiment. Details are not described herein again. In actual applications, the component radiation emission electromagnetic field risk prediction apparatus provided in the foregoing embodiment may allocate the foregoing functions to different functional modules according to a requirement, that is, divide an internal structure of the apparatus into different functional modules, to complete all or some functions described above. This is not limited herein.

An embodiment of the present invention further provides an electronic device, including: one or more processors; and a storage apparatus, configured to store one or more programs. When the one or more programs are executed by the one or more processors, the electronic device implements the component radiation emission electromagnetic field risk prediction method provided in the foregoing embodiments.

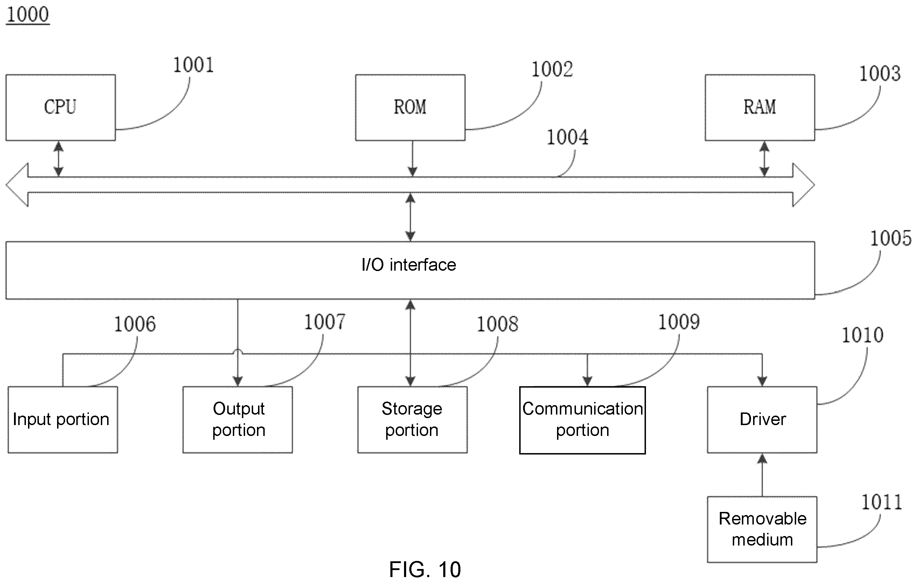

FIG. 10 is a schematic structural diagram of a computer system suitable for implementing an electronic device according to an embodiment of the present invention. It should be noted that a computer system 1000 of the electronic device shown in FIG. 10 is merely an example, and should not impose any limitation on a function and a use range of the embodiments of the present invention.

As shown in FIG. 10, the computer system 1000 includes a central processing unit (Central Processing Unit, CPU) 1001. The CPU 1001 may perform various proper actions and processing based on a program stored in a read-only memory (Read-Only Memory, ROM) 1002 or a program loaded from a storage portion 1008 to a random access memory (Random Access Memory, RAM) 1003, for example, perform the method provided in the foregoing embodiments. The RAM 1003 further stores various programs and data required for a system operation. The CPU 1001, the ROM 1002, and the RAM 1003 are connected to each other through a bus 1004. An input/output (Input/Output, I/O) interface 1005 is also connected to the bus 1004.

The following parts are connected to the I/O interface 1005: an input portion 1006 including a keyboard, a mouse, and the like; an output portion 1007 including a cathode ray tube (Cathode Ray Tube, CRT), a liquid crystal display (Liquid Crystal Display, LCD), a speaker, and the like; the storage portion 1008 including a hard disk, and the like; and a communication portion 1009 including a network interface card such as a LAN (Local Area Network, local area network) card or a modem. The communication portion 1009 performs communication processing via a network such as the Internet. A driver 1010 is also connected to the I/O interface 1005 according to a requirement. A removable medium 1011 such as a magnetic disk, an optical disc, a magneto-optical disc, or a semiconductor memory is mounted on the driver 1010 according to a requirement, so that a computer program read from the removable medium 1011 is installed into the storage portion 1008 based on a requirement.

In particular, according to the embodiments of the present invention, a process described above with reference to the flowchart may be implemented as a computer software program. For example, in an embodiment of the present invention, a computer program product is included. The computer program product includes a computer program carried on a computer-readable medium, and the computer program includes a computer program used to perform the method shown in the flowchart. In such an embodiment, the computer program may be downloaded and installed from a network through the communication portion 1009, and/or installed from the removable medium 1011. When the computer program is executed by the central processing unit (CPU) 1001, various functions defined in the system of the present invention are executed.

It should be noted that the computer-readable medium described in the embodiments of the present invention may be a computer-readable signal medium, a computer-readable storage medium, or any combination thereof. The computer-readable storage medium may be, for example, an electrical system, apparatus, or device, a magnetic system, apparatus, or device, an optical system, apparatus, or device, an electromagnetic system, apparatus, or device, an infrared system, apparatus, or device, or a semiconductor system, apparatus, or device, or any combination thereof. A more specific example of the computer-readable storage medium may include but is not limited to an electrical connection having one or more wires, a portable computer disk, a hard disk, a random access memory (RAM), a read-only memory (ROM), an erasable programmable read-only memory (Erasable Programmable Read Only Memory, EPROM), a flash memory, an optical fiber, a portable compact disk read-only memory (Compact Disc Read-Only Memory, CD-ROM), an optical storage device, a magnetic storage device, or any proper combination thereof. In the present invention, the computer-readable signal medium may include a data signal propagated in a baseband or as a part of a carrier, and carries a computer-readable computer program. The propagated data signal may be in a plurality of forms, including but not limited to an electromagnetic signal, an optical signal, or any suitable combination thereof. The computer-readable signal medium may alternatively be any computer-readable medium other than a computer-readable storage medium, and the computer-readable medium may send, propagate, or transmit a program used by or in combination with an instruction execution system, apparatus, or device. The computer program included in the computer-readable medium may be transmitted by using any proper medium, including but not limited to a wireless medium or a wired medium, or any proper combination thereof.

The flowcharts and block diagrams in the accompanying drawings show system architectures, functions, and operations that may be implemented by systems, methods, and computer program products according to various embodiments of the present invention. Each block in the flowcharts or block diagrams may represent a module, a program segment, or a part of code, and the module, the program segment, or the part of code includes one or more executable instructions for implementing a specified logical function. It should also be noted that, in some alternative implementations, a function marked in the block may also occur in a sequence different from that marked in the accompanying drawings. For example, two consecutively represented blocks may be actually executed substantially in parallel, and may sometimes be executed in a reverse order, depending on a function involved. It should also be noted that each block in the block diagrams or the flowcharts and a combination of blocks in the block diagrams or the flowcharts may be implemented by using a dedicated hardware-based system designed to perform a specified function or operation, or may be implemented by using a combination of dedicated hardware and computer instructions.

The units involved in the embodiments of the present invention may be implemented in a software manner, or may be implemented by using hardware, and the units described may also be disposed in a processor. Names of these units do not constitute a limitation on the units in a specified case.

Another aspect of the present invention further provides a computer-readable storage medium. The computer-readable storage medium stores a computer program. When the computer program is executed by a processor of a computer, the computer is enabled to perform the component radiation emission electromagnetic field risk prediction method provided in the foregoing embodiments. The computer-readable storage medium may be included in the electronic device described in the foregoing embodiments, or may exist separately, and is not assembled into the electronic device.

It should be noted that although several modules or units of the device for performing the action are mentioned in the foregoing detailed descriptions, such division is not mandatory. Actually, according to implementations of this application, features and functions of the two or more modules or units described above may be embodied in one module or unit. On the contrary, features and functions of one module or unit described above may alternatively be embodied in a plurality of modules or units.

Another aspect of this application further provides a computer program product or a computer program. The computer program product or the computer program includes computer instructions, and the computer instructions are stored in a computer-readable storage medium. A processor of a computer device reads the computer instructions from the computer-readable storage medium, and the processor executes the computer instructions, to enable the computer device to perform the component radiation emission electromagnetic field risk prediction method provided in the foregoing embodiments.

A person skilled in the art may readily understand the foregoing descriptions of the implementations. The example implementations described herein may be implemented by software, or may be implemented by software in combination with necessary hardware. Therefore, the technical solutions according to the implementations of this application may be embodied in a form of a software product. The software product may be stored in a nonvolatile storage medium (which may be a CD-ROM, a USB flash drive, or a removable hard disk) or a network, and includes several instructions, so that a computing device (which may be a personal computer, a server, a touch terminal, or a network device) performs the method according to the implementations of this application.

A person skilled in the art may easily figure out another implementation solution of this application after considering this specification and practicing the implementations disclosed herein. This application aims to cover any variation, usage, or adaptive change of this application. These variation, usage, or adaptive change follows a general principle of this application, and include common knowledge or conventional technical means in the art that are not disclosed in this application.

The foregoing embodiments merely illustrate principles and effects of the present invention, but are not intended to limit the present invention. Any person skilled in the art may modify or alter the foregoing embodiments without departing from the scope of the present invention. Therefore, all equivalent modifications or alterations completed by a person of ordinary skill in the art without departing from the technical ideas disclosed in the present invention should still be covered by the claims of the present invention.

## Claims

1. A component radiation emission electromagnetic field risk prediction method, comprising:
obtaining component simulation data, wherein the component simulation data comprises physical device data and working parameter data;
building a first simulation model based on the component simulation data, and inputting actually measured common mode current data into the first simulation model to perform spatial domain solution, to obtain a spatial domain electromagnetic field distribution and a port parameter; and
linking the spatial domain electromagnetic field distribution and the port parameter to a preset second simulation model used to perform circuit simulation, and using measured noise data and the port parameter as inputs into the second simulation model, to obtain a predicted field strength value, so as to perform risk prediction on a component radiation emission electromagnetic field.

2. The component radiation emission electromagnetic field risk prediction method according to claim 1, wherein building the first simulation model based on the component simulation data comprises:
setting a material attribute of each constituent part of a component based on the physical device data, and building a first simulation physical model;
setting a simulation port parameter based on the working parameter data, and building a first simulation port model; and
building the first simulation model based on the first simulation physical model and the first simulation port model.

3. The component radiation emission electromagnetic field risk prediction method according to claim 2, wherein setting the simulation port parameter based on the working parameter data, and building the first simulation port model comprises:
setting a component high-voltage output port as a first simulation port, setting a component low-voltage output port as a second simulation port, setting a high-voltage LISN port as a third simulation port, setting a low-voltage LISN port as a fourth simulation port, and setting a fifth simulation port between an antenna and a copper ground plate, to obtain the first simulation port model.

4. The component radiation emission electromagnetic field risk prediction method according to claim 3, wherein after the first simulation port model is obtained, the method further comprises:
inputting the actually measured common mode current data into the first simulation model to perform spatial domain solution, to obtain the spatial domain electromagnetic field distribution and the port parameter; and
based on the port parameter, inputting an actually measured high-voltage current noise source into a first circuit port, inputting an actually measured low-voltage current noise source into a second circuit port, inputting a high-voltage LISN into a third circuit port, inputting a low-voltage LISN into a fourth circuit port, and grounding a fifth circuit port to output a predicted field strength value.

5. The component radiation emission electromagnetic field risk prediction method according to any one of claims 1 to 4, further comprising:
measuring a common mode current on a high voltage direct current cable of a component and a common mode current on a low voltage harness of the component, to obtain the common mode current data; and
using the common mode current data as an initial excitation source of the first simulation model, and inputting the initial excitation source into the first simulation model.

6. The component radiation emission electromagnetic field risk prediction method according to claim 5, wherein
the first simulation model comprises a component three-dimensional equivalent model, a high voltage direct current cable equivalent model, a low voltage harness equivalent model, a high-voltage LISN equivalent model, a low-voltage LISN equivalent model, a test bench equivalent model, a ground plate equivalent model, and a receive antenna equivalent model.

7. The component radiation emission electromagnetic field risk prediction method according to claim 1, wherein before using the measured noise data and the port parameter as inputs into the second simulation model, to obtain the predicted field strength value, the method further comprises:
setting a frequency domain calculation range, and comparing the frequency domain calculation range with an actually measured excitation source;
resetting the frequency domain calculation range if the frequency domain calculation range is inconsistent with the actually measured excitation source, and
determining that the frequency domain calculation range is set correctly if the frequency domain calculation range is consistent with the actually measured excitation source.

8. The component radiation emission electromagnetic field risk prediction method according to claim 1, wherein using the measured noise data and the port parameter as inputs into the second simulation model comprises:
perform simplification of the measured noise data, to obtain simplified data; and
if an interference feature of the simplified data is inconsistent with an interference feature of the measured noise data, obtaining new measured noise data to perform the simplification; and
inputting the simplified data into the second simulation model if the interference feature of the simplified data is consistent with the interference feature of the measured noise data.

9. The component radiation emission electromagnetic field risk prediction method according to claim 1, further comprising:
obtaining radiation emission data at a distance of a first preset value from a component test rack;
using the measured noise data and the port parameter as an excitation source of the second simulation model, to obtain the predicted field strength value; and
performing comparison verification on the radiation emission data and the predicted field strength value, to perform risk prediction on the component radiation emission electromagnetic field.

10. A component radiation emission electromagnetic field risk prediction apparatus, comprising:
an obtaining module, configured to obtain component simulation data, wherein the component simulation data comprises physical device data and working parameter data;
a first simulation module, configured to: build a first simulation model based on the component simulation data, and input actually measured common mode current data into the first simulation model to perform spatial domain solution, to obtain a spatial domain electromagnetic field distribution and a port parameter; and
a second simulation module, configured to: link the spatial domain electromagnetic field distribution and the port parameter to a preset second simulation model used to perform circuit simulation, and use measured noise data and the port parameter as inputs into the second simulation model, to obtain a predicted field strength value, so as to perform risk prediction on a component radiation emission electromagnetic field.

11. An electronic device, comprising:
one or more processors; and
a storage apparatus, configured to store one or more programs, wherein when the one or more programs are executed by the one or more processors, the electronic device is adapted to implement the component radiation emission electromagnetic field risk prediction method according to any one of claims 1 to 9.

12. A computer-readable storage medium, wherein the computer-readable storage medium stores a computer program, and when the computer program is executed by a processor of a computer, the computer is enabled to perform the component radiation emission electromagnetic field risk prediction method according to any one of claims 1 to 9.
